# DEMANDE DE BREVET EUROPEEN

(11) **EP 2 606 821 A1**
(43) Date de publication de la demande: **26.06.2013**
(21) Numéro de dépôt: 11194621.6
(22) Date de dépôt: 20.12.2011
(51) Int. Cl.: A61B 5/103, G01L 1/14, G01L 9/00, G01L 9/10

(54) **Capteur de pression inductif**

(71) Demandeur: Rocklinger, Marc, 74380 Bonne (FR); Vacherand, Pascal, 74380 Lucinges (FR); Brönnimann, Frédéric, 1261 Le Vaud (CH)
(72) Inventeur: Rocklinger, Marc, 74380 Bonne (FR); Vacherand, Pascal, 74380 Lucinges (FR); Brönnimann, Frédéric, 1261 Le Vaud (CH)
(74) Mandataire: Bugnion Genève

(57) **Abrégé**

Capteur de pression (2) comprenant, empilées les unes sur les autres, au moins une couche cible (305) en un matériau électriquement conducteur, au moins une couche élastiquement déformable (304) en un matériau compressible et une bobine (303) comprenant des moyens de connexion permettant de connecter ladite bobine à une alimentation en courant.

## Description

### DOMAINE TECHNIQUE DE L'INVENTION

La présente invention concerne le domaine des capteurs de pression. L'invention porte en particulier sur un capteur de pression comprenant, empilées les unes sur les autres, au moins une couche cible en un matériau électriquement conducteur, au moins une couche élastiquement déformable en un matériau compressible et une bobine comprenant des moyens de connexion permettant de connecter ladite bobine à une alimentation en courant.

### ETAT DE LA TECHNIQUE ANTERIEURE

Les capteurs de pression sont traditionnellement des capteurs capacitifs, résistifs ou piézo-électriques. De tels capteurs sont généralement constitués de matériaux en céramique (diélectriques) ou en alliages métalliques. Le problème majeur de tels capteurs provient de leur fragilité qui induit une durée de vie limitée.

### EXPOSÉ DE L'INVENTION

Un premier but de l'invention est de fournir un capteur plus solide et dont la durée de vie est accrue.

Un deuxième but de l'invention est de fournir un capteur de pression dont la sensibilité de mesure de la pression est accrue.

Selon l'invention, le capteur de pression comprend, empilées les unes sur les autres, au moins une couche cible en un matériau électriquement conducteur, au moins une couche élastiquement déformable en un matériau compressible et une bobine comprenant des moyens de connexion permettant de connecter ladite bobine à une alimentation en courant, la bobine comportant une première couche conductrice en un matériau électriquement conducteur, une deuxième couche conductrice en un matériau électriquement conducteur et une couche isolante en un matériau électriquement isolant, la première couche conductrice étant connectée électriquement à la deuxième couche conductrice et la couche isolante étant disposée entre la première couche conductrice et la deuxième couche conductrice.

Selon l'invention, la première couche conductrice peut consister en une première piste conductrice.

Selon l'invention, la première piste conductrice peut présenter une forme spiralée.

Selon l'invention, la deuxième couche conductrice peut consister en une deuxième piste conductrice.

Selon l'invention, la deuxième piste conductrice peut présenter une forme spiralée.

Selon l'invention, la première couche conductrice et la deuxième couche conductrice peuvent avoir une épaisseur comprise entre 7 et 38 micromètres.

Selon l'invention, la couche élastiquement déformable peut avoir une épaisseur comprise entre 250 et 1000 micromètres.

Selon l'invention, la couche isolante peut avoir une épaisseur comprise entre 25 et 100 micromètres.

Selon l'invention, le matériau électriquement conducteur peut être du cuivre.

Selon l'invention, le matériau électriquement isolant peut être un polyester ou un polyimide.

Selon l'invention, le matériau compressible peut être une mousse élastomère.

Selon l'invention, ledit capteur peut comprendre une première couche de protection disposée extérieurement contre la couche cible et/ou une deuxième couche de protection disposée extérieurement contre la deuxième couche conductrice.

Selon l'invention, le capteur peut être de forme cylindrique.

Selon l'invention, la hauteur du capteur peut être inférieure à 2mm.

Selon l'invention, le diamètre du capteur peut être compris entre 5mm et 20mm.

### DESCRIPTION DES FIGURES

D'autres caractéristiques et avantages de l'invention ressortiront à la lecture de la description qui suit, en référence aux figures annexées, dans laquelle :
- la figure 1 montre une vue en perspective d'un capteur de pression selon l'invention ;
- la figure 2 montre schématiquement la structure d'un capteur de pression selon l'invention.

### DESCRIPTION DÉTAILLÉE

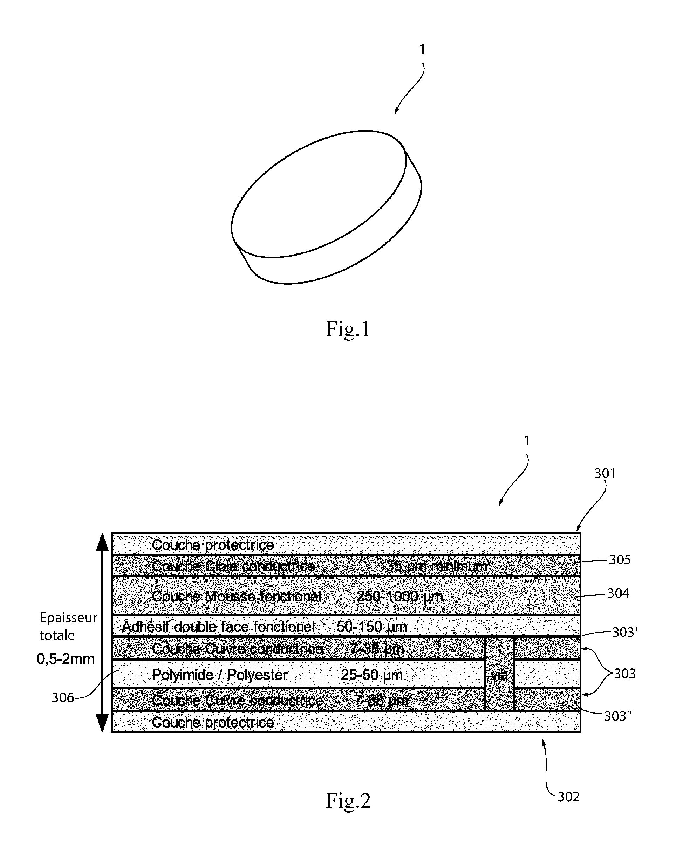

Le capteur de pression 1 représenté à la figure 1 est de forme cylindrique. Le capteur de pression peut toutefois également avoir une forme parallélépipédique. De préférence, l'épaisseur du capteur ne dépasse pas 2mm et son diamètre est compris entre 5mm et 20mm. Le capteur de pression selon l'invention est évidemment destiné à être utilisé avec un module électronique (non représenté) apte à lui fournir l'énergie nécessaire à son fonctionnement et également capable d'interpréter et d'analyser les grandeurs électriques (tension et/ou intensité du courant) véhiculées en sortie du capteur pour finalement déterminer l'amplitude d'une force de pression éventuellement appliquée sur le capteur. Le capteur de pression selon l'invention peut avantageusement être implanté dans une semelle pour servir ainsi à mesurer une pression plantaire.

La figure 2 montre une vue selon une coupe transversale du capteur de pression selon l'invention. Comme on peut le voir, la structure du capteur se présente sous la forme d'un « sandwich » de plusieurs couches de différents matériaux empilées les unes sur les autres. Le capteur 1 comprend une première face 301 et une deuxième face 302 planes entre lesquelles sont disposées les couches.

Le capteur 2 est de type inductif car il comprend en particulier une bobine 303. La bobine 303 est formée de deux couches électriquement conductrices (303', 303") réalisées par exemple sous forme de deux enroulements de pistes conductrices reliés électriquement l'un à l'autre. Entre les deux couches conductrices (303', 303") est disposée une couche isolante 306 plane, réalisée dans un matériau électriquement non conducteur. La couche isolante 306 peut par exemple être constituée d'un polyimide ou d'un polyesther ou tout autre matériau électriquement isolant. L'épaisseur de la couche isolante 306 est comprise entre 25 et 100 µm.

Les pistes conductrices peuvent par exemple présenter une forme spiralée. La bobine 303 est de préférence fabriquée selon les techniques de fabrication des circuits imprimés flexible (Flexible Printed Circuit Boards ou FPCB). Chaque piste conductrice peut par exemple être en cuivre. Les enroulements des pistes conductrices sont tels que le courant électrique circule dans le même sens dans les deux enroulements. L'épaisseur de chacune des couches conductrices 303', 303" est de préférence comprise entre 7 et 38 µm. Les pistes conductrices sont reliées électriquement entre elles par exemple au travers d'un « via » traversant la couche plane 306. Un « via » se présente sous la forme d'un trou métallisé reliant les pistes conductrices entre elles.

La structure en deux couches conductrices de la bobine 303 permet de doubler le champ magnétique et ainsi d'augmenter la sensibilité du capteur 2. De plus, les deux enroulements sont isolés l'un de l'autre par la couche plane 306 et peuvent être rapprochés le plus possible pour annuler l'effet magnétique/inductance des pistes.

Le capteur comprend de plus une couche élastiquement déformable 304 réalisée en un matériau élastiquement compressible et une couche cible 305, ou cible, réalisée en un matériau électriquement conducteur. Le matériau de la couche élastiquement déformable 304 disposée entre la bobine bicouche et la cible 305 est perméable au champ magnétique. Il peut être formé de matériaux plastiques, par exemple une mousse élastomère.

Finalement, une couche de protection est disposée sur chaque face du capteur, l'une sur la couche cible 305 et l'autre sous la deuxième couche conductrice 303", afin d'empêcher une oxydation éventuelle. Ces couches de protection ne sont toutefois pas obligatoires et leur présence dépend de l'intégration du capteur dans son milieu.

La mousse élastomère de la couche élastiquement déformable 304 est soit autocollante, soit déposée à l'aide d'un adhésif double face de transfert. Le tout est laminé sous pression sur une face. La couche cible 305 est soit déposée par jet d'une encre conductrice ou collée et laminée sur la mousse. Selon une variante d'exécution, on peut ajouter une deuxième couche élastiquement déformable et une deuxième couche cible en dessous de la bobine 303.

Le fonctionnement du capteur de pression selon l'invention est le suivant.

Lorsqu'un courant électrique circule dans la bobine bicouche 303, il se produit un champ magnétique qui est dévié par la couche cible 305. Le capteur 2 possède donc une inductance propre. Lorsqu'une pression est exercée sur le capteur 2, cette pression induit une compression de la couche élastiquement déformable 304 et ceci a pour conséquence de rapprocher la bobine 303 et la cible 305. Ainsi, sous l'action d'une pression positive ou négative, la bobine 303 et la cible 305 se rapprochent ou s'éloignent et ceci contribue donc à modifier le chemin du flux magnétique. Ceci a pour conséquence de faire varier l'inductance de la bobine 303. Cette inductance qui varie en fonction de la distance entre la bobine 303 et la cible 305 peut être mesurée au travers de la tension aux bornes de la bobine bicouche 303 (appelée force électromotrice) et, sur la base de la variation de l'inductance induite par une pression exercée sur une des faces du capteur, il est possible de déterminer la pression exercée sur le capteur.

## Revendications

1. Capteur de pression (2) comprenant, empilées les unes sur les autres, au moins une couche cible (305) en un matériau électriquement conducteur, au moins une couche élastiquement déformable (304) en un matériau compressible et une bobine (303) comprenant des moyens de connexion permettant de connecter ladite bobine à une alimentation en courant, **caractérisé en ce que** la bobine (303) comporte une première couche conductrice en un matériau électriquement conducteur (303'), une deuxième couche conductrice (303") en un matériau électriquement conducteur et une couche isolante (306) en un matériau électriquement isolant, la première couche conductrice (303') étant connectée électriquement à la deuxième couche conductrice (303") et la couche isolante étant disposée entre la première couche conductrice (303') et la deuxième couche conductrice (303").

2. Capteur de pression selon la revendication 1, **caractérisé en ce que** la première couche conductrice (303') consiste en une première piste conductrice.

3. Capteur de pression selon la revendication 2, **caractérisé en ce que** la première piste conductrice présente une forme spiralée.

4. Capteur de pression selon la revendication 1, **caractérisé en ce que** la deuxième couche conductrice (303") consiste en une deuxième piste conductrice.

5. Capteur de pression selon la revendication 4, **caractérisé en ce que** la deuxième piste conductrice présente une forme spiralée.

6. Capteur de pression selon l'une des revendications précédentes, **caractérisé en ce que** la première couche conductrice (303') et la deuxième couche conductrice (303") ont une épaisseur comprise entre 7 et 38 micromètres.

7. Capteur de pression selon l'une des revendications précédentes, **caractérisé en ce que** la couche élastiquement déformable (304) a une épaisseur comprise entre 250 et 1000 micromètres.

8. Capteur de pression selon l'une des revendications précédentes, **caractérisé en ce que** la couche isolante (306) a une épaisseur comprise entre 25 et 100 micromètres.

9. Capteur de pression selon l'une des revendications précédentes, **caractérisé en ce que** le matériau électriquement conducteur est du cuivre.

10. Capteur de pression selon l'une des revendications précédentes, **caractérisé en ce que** le matériau électriquement isolant est un polyester ou un polyimide.

11. Capteur de pression selon l'une des revendications précédentes, **caractérisé en ce que** le matériau compressible est une mousse élastomère.

12. Capteur de pression selon l'une des revendications précédentes, **caractérisé en ce que** ledit capteur comprend une première couche de protection disposée extérieurement contre la couche cible (305) et/ou une deuxième couche de protection disposée extérieurement contre la deuxième couche conductrice (303").

13. Capteur de pression selon l'une des revendications précédentes, **caractérisé en ce que** le capteur est de forme cylindrique.

14. Capteur de pression selon la revendication précédente, **caractérisé en ce que** la hauteur du capteur est inférieure à 2mm.

15. Capteur de pression selon l'une des revendications 13 ou 14, **caractérisé en ce que** le diamètre du capteur est compris entre 5mm et 20mm.
